# EUROPÄISCHE PATENTSCHRIFT

(11) **EP 2 397 848 B1**
(45) Veröffentlichungstag und Bekanntmachung des Hinweises auf die Patenterteilung: **30.05.2018**
(21) Anmeldenummer: 11169572.2
(22) Anmeldetag: 10.06.2011
(51) Int. Cl.: G01N 29/14, G01N 29/07, G01N 33/38, G01N 29/11, G01N 17/00

(54) **Kontinuierliche Dehnungsmessung bei Prüfung des Einflusses der Alkali-Kieselsäure-Reaktion auf Gefügeveränderungen in Betonen**
Continuous strain measurement when testing the influence of the alkali-silica reaction on microstructural changes in concrete
Mesure de déformation continue lors du contrôle de l'influence de la réaction silice- alcalis sur des modifications de microstructures dans des bétons

(30) Priorität: 18.06.2010 DE 102010017468
(43) Veröffentlichungstag der Anmeldung: 21.12.2011
(73) Patentinhaber: BAM Bundesanstalt für Materialforschung und -prüfung, 12205 Berlin (DE)
(72) Erfinder: Weise, Frank, 12526 Berlin (DE); Pirskawetz, Stephan, 10119 Berlin (DE); Maier, Bärbel, 12359 Berlin (DE); Voland, Katja, 12163 Berlin (DE)
(74) Vertreter: Zimmermann & Partner Patentanwälte mbB

(56) Entgegenhaltungen:
- DE-A1- 3 928 130
- DE-C1- 19 626 111
- US-A1- 2007 056 383

## Beschreibung

Die vorliegende Erfindung betrifft zerstörungsfreie Prüfverfahren für Probekörper unter simulierten Bedingungen einer beschleunigten Alterung. Insbesondere betrifft die vorliegende Erfindung die Beurteilung innerer Gefügeveränderungen in Betonen unter Bedingungen, die eine Alkali-Kieselsäure-Reaktion (AKR) provozieren. Dabei wird eine Dehnungsmessung und eine Schallemissionsmessung und/oder eine Ultraschallgeschwindigkeitsmessung kontinuierlich ausgeführt.

Die Begriffe Alkali-Kieselsäure, Alkali-Silikat- und Alkali-Karbonat-Reaktion werden häufig unter dem Begriff Alkali-Aggregat-Reaktion zusammengefasst. Darunter versteht man eine chemische Reaktion alkaliempfindlicher Bestandteile bestimmter Gesteinskörnungen und den Alkalihydroxiden. Die genannten Reaktionen unterscheiden sich lediglich in der Art der alkaliempfindlichen Gesteinskörnung. Bei der Alkali-Kieselsäure-Reaktion (AKR), auch Alkali-Silica-Reaktion genannt, handelt es sich um amorphe bzw. schlecht kristalline SiO₂ (Kieselsäure). Das Quellvermögen der dabei gebildeten Gele kann eine Rissbildung und damit einhergehende Schädigung von Betonkonstruktionen unter Feuchteeinfluss, langfristig die völlige Erosion des Betons bewirken. Zusätzlich zum Alkalieintrag über den Zement, führen von außen in den Beton eindringende Salze zur AKR und den damit einhergehenden Schäden.

Im Rahmen der Eignungsprüfung von Gesteinskörnungen bietet neben dem Schnellprüfverfahren bzw. Mörtelschnelltest der Betonversuch mit Nebelkammerlagerung bzw. alternativ der 60 °C-Betonversuch über Wasser die Möglichkeit, das Gefährdungspotential durch die AKR einzuschätzen und so Folgeschäden vorzubeugen. Entsprechende Prüfverfahren sind international üblich und beispielsweise in Deutschland gemäß Alkali-Richtlinie ("Vorbeugende Maßnahmen gegen schädigende Alkalireaktion im Beton. (Alkali-Richtlinie" - Fassung Februar 2007, Herausgeber: Deutscher Ausschuss für Stahlbeton (DAfStb)) vorgeschrieben. Weitere Prüfmethoden sind RILEM AAR-3 und 4, der US-Standard ASTM C1293, die französische Prüfvorschrift NF P 18-454.

Gemäß einem festgelegten Betonversuch mit Nebelkammerlagerung bei 40 °C wird einerseits die Längenänderung von Beton-Prismen sowie andererseits die Rissweitenentwicklung auf der Oberfläche von Betonwürfeln als Schädigungsindikator für die AKR herangezogen. Die Probekörper werden in einer Nebelkammer bei 40 °C und 100% Luftfeuchte gelagert. Die Länge des Probekörpers wird nach erster Messung am Tag "0" (direkt nach dem Ausschalen der einen Tag zuvor hergestellten Probekörper) an den darauf folgenden Tagen 1, 7 und 28 und anschließend aller weiteren 28 Tage über die Dauer von insgesamt 252 Tagen bestimmt.

Im Betonversuch bei 60 °C über Wasser (60 °C-Betonversuch) werden prismatische Probekörper bei 60 °C und mindestens 98 % Luftfeuchte in einer AKR-Truhe (Reaktor) gelagert. In diesem Fall erfolgt die Bestimmung der Längenänderung von Anfang an alle 28 Tage. Dazu werden die Probenbehälter 24 Stunden vor der Messung aus der AKR-Truhe entnommen und bei 20°C und mindestens 95 % relative Luftfeuchte gelagert.

Die bisherige Vorgehensweise eignet sich nur bedingt zur ganzheitlichen Erfassung der ablaufenden inneren Gefügeveränderungen infolge der Hydratation und Schädigung. Damit ist eine differenzierte Betrachtung der Rissbildung und des sich vermutlich anschließenden Füllens der Risse mit Alkalisilikagel während des Schädigungsprozesses gleichfalls nicht möglich.

Die Druckschriften DE 196 26 111 und DE 39 28 130 beschreiben ein Verfahren zur Prüfung des Frost-Tau-Widerstands von Festkörpern, deren Beanspruchungsfläche in einer Prüfflüssigkeit ruht. Vorgegebene Temperatur-Zeit-Profile simulieren einen Frost-Tau-Wechsel. Die Druckschrift US 2007/0056383 beschreibt eine Vorrichtung zur Herstellung eines Prüfkörpers aus einer Zementmischung und ein Verfahren mit einer zylindrischen Testzelle einer Druckkammer zur Bestimmung der mechanischen Eigenschaften der Zementmischung für die Befestigung von Bohrlöchern.

Vor diesem Hintergrund stellt die vorliegende Erfindung eine Prüfmethodologie bereit, welche kontinuierlich und/oder in vorgegebenen Zeitabständen Hydratations- und Schädigungsprozesse in Betonen bei AKR-provozierender Lagerung erfasst und eine Überlagerung mit einer zusätzlichen thermisch und/oder hygrisch induzierten Rissbildung vermeidet.

Die beschriebene Prüfmethodologie eignet sich auch zur Verfolgung von Rissbildungsprozessen, die durch andere Schädigungsprozesse in mineralischen Baustoffen induziert werden. Das betrifft beispielsweise neben mechanisch und thermomechanisch induzierten Schädigungsprozessen auch die Rissbildung durch behindertes Schwinden und Sulfattreiben.

Bereitgestellt wird ein automatisches Messverfahren und eine Vorrichtung zur kontinuierlichen Erfassung innerer Gefügeveränderungen beispielsweise in Betonen infolge von Hydratation sowie Riss- und Gelbildung in Probekörpern durch kontinuierliche Messung der Längenänderung und der Schallemissionsaktivität und/oder der Ultraschalllaufzeit. Dabei werden innere Gefügeveränderungen während der AKR-provozierenden Lagerung kontinuierlich in situ erfasst. Die Klimatisierung der Probekörper muss demzufolge nicht unterbrochen werden. Dadurch können verfälschende Einflüsse durch Abkühlung und eventuelle zusätzliche Feuchteabgabe vermieden werden.

Das zur Erfassung der Rissbildung verwendete Messprinzip basiert auf der Erfassung der mit den Rissbildungen einhergehenden ruckartigen Materialverschiebungen. Eine derartige Materialverschiebung pflanzt sich als elastische Welle, also als Ultraschallwelle, vom Riss ausgehend im Material fort. Mit an der Oberfläche des Probekörpers angekoppelten Schallemissionssensoren, sogennannten SE-Sensoren, können diese Ultraschallwellen in elektrische Signale umgewandelt werden. Die Geschwindigkeit, Frequenz und das Abklingverhalten der Ultraschallwelle hängen dabei von den elastischen Materialeigenschaften ab.

Vorteile der hier beschriebenen Vorrichtung bzw. des beschriebenen Messverfahrens ergeben sich aus der kontinuierlich erfolgenden Schallemissionsanalyse in Kombination mit periodischen Ultraschallmessungen. Das erlaubt erstmals Aussagen über den zeitlichen Verlauf der Hydratations-, Riss- und Gelbildungsprozesse in Betonen während der AKR-provozierenden Lagerung.

Besonders vorteilhaft dabei ist, dass der Schädigungsverlauf und somit das Prüfergebnis nicht durch die Entnahme der Proben aus dem jeweiligen Klima und eine mit der Entnahme und manuellen Messung verbundenen Abkühlung und teilweisen Austrocknung der Proben beeinflusst wird.

Insbesondere liefert der kombinierte Einsatz der Schall-Emissions-Analyse (SEA) und der Ultraschalllaufzeitmessungen bei in der Nebelkammer bei 40 °C gelagerten Würfeln zusätzlich zu der Rissweitenmessung wertvolle Schädigungsindikatoren. Außerdem ist die bisherige Rissweitenbestimmung eher ungenau.

Insgesamt erlaubt die beschriebene Prüfmethodologie mit der beschriebenen Messvorrichtung eine kontinuierliche on-line Erfassung der Schadensentwicklung unter gleichzeitiger Minimierung des Messaufwandes unter weitestgehendem Ausschluss subjektiver Faktoren.

Insbesondere wird die Entnahme der Probekörper aus der Nebelkammer bzw. dem Reaktor und die damit verbundene Abkühlung und partielle Austrocknung der Proben vermieden. Ebenso der daraus resultierende Temperatur- und Feuchtegradient, der Eigenspannungen zur Folge haben kann, die dann den AKR-induzierten Schädigungsprozess überlagern.

Weiterhin ist der Zeitraum zwischen zwei Messungen im Vergleich zu vorbekannten Methoden beliebig kurz. Dies stellt besonders für den 60 °C-Betonversuch einen erheblichen Vorteil dar, da bei stark alkaliempfindlichen Gesteinskörnungen die Reaktion schon nach einem Monat weitgehend beendet sein kann. Aussagen über den Verlauf der Schädigung können daher gut dokumentiert werden.

Damit kann der Probendurchsatz gesteigert, vor allem aber die Zuverlässigkeit erhobener Befunde verbessert werden.

Die vorliegende Erfindung wird nachfolgend beispielhaft an Hand von Figuren erläutert. Dabei zeigt:
- Fig. 1: den schematischen Mess-Aufbau;
- Fig. 2: den schematischen Aufbau des Rahmens mit Sensoren für die kontinuierliche Dehnungsmessung und Schallemissions-Analyse an einem Probekörper;
- Fig. 3: Ergebnisse der kontinuierlichen Messung von Ultraschallgeschwindigkeit (US), Schallemissionsaaktivität (SEA) und diskontinuierlichen Bestimmung der Rissweite (RW) eines in der Nebelkammer bei 40 °C eingelagerten Würfels mit 300 mm Kantenlänge am Beispiel von Grauwackesplitt-Beton bei w/z = 0,35.
- Fig. 4: Ergebnisse der kontinuierlichen Messung von Ultraschallgeschwindigkeit (US), Schallemissionsaktivität (SEA) und Längenänderung (ε) von im Reaktor bei 60 °C eingelagerten Zylindern (Ø 70 mm, L 280 mm) am Beispiel von Grauwackesplitt-Beton bei w/z = 0,35.
- Fig. 5: Gegenüberstellung der Ergebnisse der kontinuierlichen und manuellen (●) Messung der Längenänderung von im Reaktor bei 60 °C eingelagerten Zylindern (Ø 70 mm, L 280 mm) am Beispiel von Grauwackesplitt-Beton bei w/z = 0,35.
- Fig. 6: Gegenüberstellung der Ergebnisse der kontinuierlichen und manuellen (●) Messung der Längenänderung von im Reaktor bei 60 °C eingelagerten Zylindern (Ø 70 mm, L 280 mm) am Beispiel von Beton mit Kiesedelsplitt vom Oberrhein bei w/z = 0,35.
- Fig. 7: Gegenüberstellung der Ergebnisse der kontinuierlichen und manuellen (●) Messung der Längenänderung von im Reaktor bei 60 °C eingelagerten Zylindern (Ø 70 mm, L 280 mm) am Beispiel von gebrochenem Rhyolith-Beton bei w/z = 0,35.

Bei der Verwendung von eigens an die Bedingungen üblicher Prüfkammern für Beton-Probekörper (40 °C Nebelkammer, 60 °C-Betonversuch) angepasster Messfühler zur kontinuierlichen Datenaufnahme wurden überraschende Abweichungen der Messkurven gegenüber den nach bekannten Messverfahren diskontinuierlich aufgenommenen Dehnungsverläufen festgestellt, die auf Grund vorliegender Erfahrungen nicht zu erwarten waren.

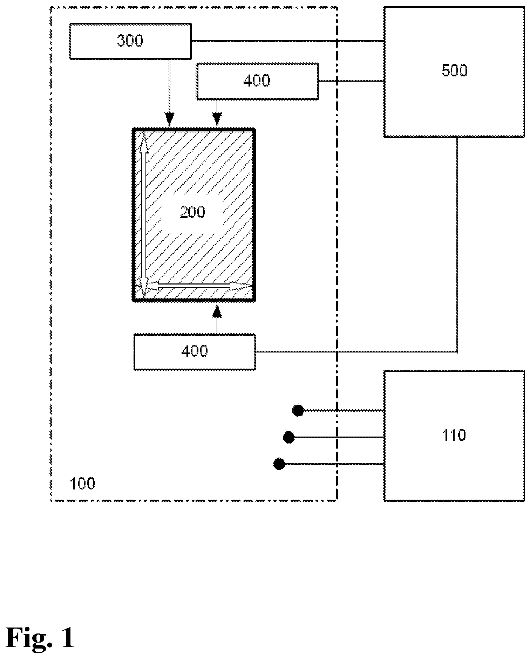

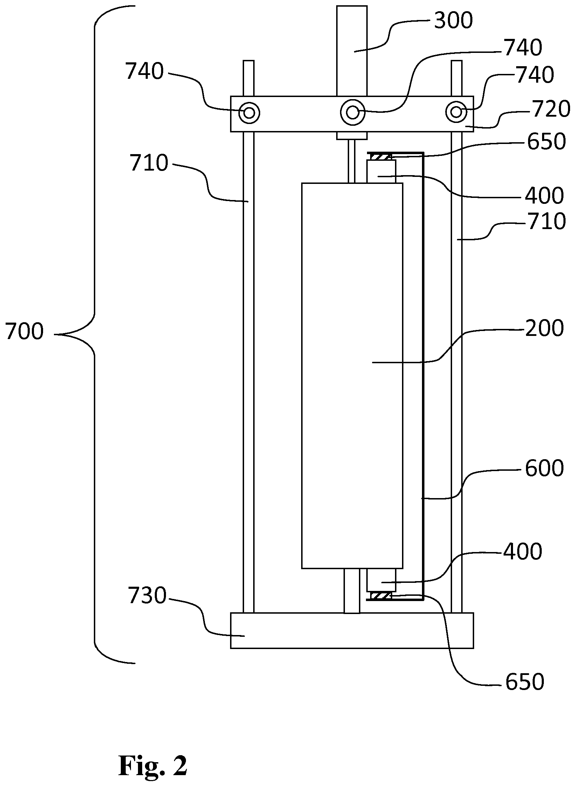

Der Messaufbau umfasst eine geeignet thermisch isolierte und klimatisierte Kammer, eine Probehalterung zur Aufnahme der Probe sowie Sensoren die mit einem Steuergerät bzw. einer Steuer- und Auswerteeinheit verbunden sind. Die Vorrichtung für das beschriebene Messverfahren ist schematisch in Figur 1 dargestellt. Dabei ist die klimatisierte Kammer 100 und der Probekörper 200, dessen Verhalten unter den Prüfbedingungen erfasst werden soll, mit den Sensoren 300, 400 gezeigt. Details einer für das beschriebene Messverfahren geeigneten Vorrichtung sind in Figur 2 gezeigt.

Die Probenhalterung bzw. der Messrahmen 700 besteht aus einem Material, welches gegen das Klima (60 °C bzw. 40 °C und eine relative Luftfeuchte > 98 %) dauerhaft beständig ist. Ein geeignetes Material ist beispielsweise V4A (Werkstoffnummer 1.4571 - X6CrNiMoTi17-12-2). Der Rahmen 700 ist so dimensioniert, dass er sich auch bei langzeitiger mechanischer Beanspruchung durch die Probe nicht deformiert. Nach einer Ausführungsform kann der Rahmen 700 auch in den für die Lagerung bei 60 C° vorgesehenen Probenbehälter 100 eingehängt werden. Für eine Lagerung bei 40 °C und ≥ 99% Luftfeuchte in einer Nebelkammer 100 kann zusätzlich ein gesondertes Gestell verwendet werden, in das eine Vielzahl von mit Proben bestückten Rahmen 700 eingehängt werden kann. Ein Rahmen 700 kann beispielsweise auch so konstruiert sein, dass er in der Nebelkammer 100 ohne ein gesondertes Gestell stehen kann. Dazu wird der Rahmen 700 mit einer angepassten Basis 730, beispielsweise mit einer Fußplatte ausgestattet.

Werden Schallemissionsmessungen durchgeführt, so ist der Rahmen vorteilhafterweise akustisch vom Probenbehälter bzw. vom Gestell entkoppelt. Dafür werden zwischen den Probenbehälter bzw. das Gestell und den den Rahmen haltenden Bügel Gummipuffer gelegt. Ein geeignetes Material für die Puffer ist beispielsweise EPDM (Ethylen-Propylen-Dien-Kautschuk). Ebenso können andere Materialien, die zur akustischen Entkopplung geeignet und gegen das Klima dauerhaft beständig sind, eingesetzt werden. Vorteilhafterweise quillt für den Puffer geeignetes Material unter der Bewitterung nicht.

Der obere Querbalken des Rahmens 720, bzw. das Querhaupt 720, ist auf den Säulen 710 verschiebbar und mit geeigneten Befestigungsmitteln 740 an diesen fixierbar. Dazu können beispielsweise durchgehende Bohrungen oder seitliche Schlitze, die die Säulen 710 aufnehmen, vorgesehen sein. Geeignete Befestigungsmittel 740 für die Fixierung sind beispielsweise Schrauben. Alternativ kann das Querhaupt 720 aber auch an den Säulen 710 einrasten oder mit einer Spannvorrichtung an diesen fixiert sein. So ist eine Anpassung des Rahmens 700 an verschiedene Höhen unterschiedlicher Probenkörper 200 möglich. Der Wegaufnehmer 300 sollte im oberen Querhaupt 720 des Rahmens 700 vertikal verschiebbar sein. Er ist mit einem Befestigungsmittel 740, beispielsweise einer Schraube oder einer Spannvorrichtung am Querhaupt 720 fixierbar. Für die Befestigung des Wegaufnehmers 300 am Querhaupt 720 und für die Befestigung des Querhaupts 720 an den Säulen 710 des Rahmens 700 können unterschiedliche Befestigungsmittel verwendet werden.

Die Möglichkeit, den Wegaufnehmer 300 am Querhaupt 720 nach jeweiligem Erfordernis zu fixieren, ermöglicht die Justierung der Lage des Wegaufnehmers 300 in der Mitte seines Messbereiches. So kann die beim Beginn der Bewitterung zunächst unbekannte Längenänderung einer unbekannten Probe ohne zwischenzeitliche Nachjustierung optimal erfasst werden.

Neben der automatisch erfassten Längenänderung des klimatisierten Probekörpers 200 mit Wegaufnehmern 300 wird die zeitliche Änderung der Ultraschallgeschwindigkeit und die akustische Emission quasi kontinuierlich aufgezeichnet. Dadurch können Aussagen zur Rissbildung im Probekörper 200 getroffen werden.

Ein geeigneter Wegaufnehmer widersteht dem Klima dauerhaft. Geeignete Wegaufnehmer sollten deshalb nicht korrodieren. Ihr Messsignal weist auch über lange Zeiträume (> 12 Monate) keine Drift auf. Das heißt, dass sich die Anzeige des Wegaufnehmers nicht ändert, wenn der Probekörper oder ein Referenzstab keine Dehnung aufweist. Geeignete Wegaufnehmer erlauben beispielsweise die Messung einer Dehnung oder Schrumpfung der Probe mit einer Genauigkeit von ± 2 µm. Erreicht wird dies beispielsweise mit induktiven Wegaufnehmern mit einem Messbereich von ± 1 mm und einem Linearitätsfehler von 0,1 % bezogen auf den jeweiligen Gesamtmessweg.

Ist der Linearitätsfehler des Wegaufnehmers 300 größer, so kann er auch mit einer ausreichend genauen Kalibriervorrichtung über die dem Fachmann bekannte Verwendung einer Linearisierungsfunktion kalibriert werden. Es können alle Typen von Wegaufnehmern verwendet werden, welche die genannten Bedingungen erfüllen und die in den Messrahmen eingebaut werden können. Beispiele für geeignete Wegaufnehmer sind z.B. kapazitive, resistive, oder optische Wegaufnehmer.

Eine Besonderheit des beschriebenen Messverfahrens besteht in der Kombination des Einsatzes von Wegaufnehmern 300 und der Messung der Schallgeschwindigkeit sowie der Registrierung von Rissbildungen mit Hilfe von zwei einander gegenüber positionierten Schallemissionssensoren, bzw. Aufnehmern 400.

Die Schallemissionssensoren bzw. Aufnehmer 400 sollten dem Klima für die Dauer der Messungen dauerhaft widerstehen. Als besonders geeignet erwiesen sich komplett wasserdichte, piezoelektrische Sensoren mit einem Gehäuse aus V4A (Fa. Vallen, Typ VS 150-K). Die Aufnehmer 400 müssen den sonst für die Schallemissionsanalyse an Beton verwendeten Aufnehmern entsprechen. Üblicherweise liegt der Arbeitsbereich geeigneter Aufnehmer im Frequenzbereich von 20 kHz bis 1000 kHz.

Für den Einsatz zur Messung der Ultraschallgeschwindigkeit sind Schallemissionssensoren bzw. Aufnehmer 400 als Ultraschallpulsgeber verwendbar. Dafür ist das verwendete Steuergerät für die Schallemission, das Bestandteil der Steuer- und Auswerteeinheit 500 ist, in der Lage, Spannungspulse bis 400 V_{PP} zu generieren und diese Pulse durch die mit dem Steuergerät verbundenen Vorverstärker an den Schallemissionssensor 400 zu geben.

Die Schallemissionssensoren bzw. Aufnehmer 400 sind an den Stirnflächen der Probekörper über einen geeignet geformten Bügel 600 befestigt. Ein derartiger Bügel kann beispielsweise aus V4A-Blech bestehen. Dies gewährleistet eine dauerhaft gleichmäßige Andruckkraft der Sensoren an die Stirnflächen der Proben. Die Bügel sollten die Proben an keiner Stelle berühren. Die Sensoren sind durch geeignete Gummipuffer, beispielsweise EPDM akustisch von den Bügeln entkoppelt. Die Sensoren können auch mit einem elastischen Band, beispielsweise einem Gummiband oder einem anderen geeigneten Rahmen oder beispielsweise mit einem geeigneten Kleber an den Proben befestigt sein.

Die mit dem Wegaufnehmer 300 und den Schallemissionssensoren bzw. Aufnehmern 400 durchgeführten Messungen an einem Probenkörper erfolgen in einem frei wählbaren oder einem fest eingestellten Zeittakt. Beispielsweise sind die Zeittakte der unterschiedlichen Messungen aufeinander abgestimmt.

1. Dehnungsmessungen: Die Dehnung der betreffenden Probe wird beispielsweise alle 15 Minuten automatisch gemessen und der ermittelte Wert wird gespeichert. Messungen können aber ebenso wöchentlich, täglich, stündlich, oder beispielsweise auch im Minuten-Takt erfolgen. Höhere Messraten sind üblicherweise nicht erforderlich, da die Prozesse langsam ablaufen und die auszuwertende Datenmenge andernfalls sehr groß wird. Für die hier beschriebenen Anwendungen zur Prüfung der AKR an Betonen werden Messungen im 1-Stunden bis 4-Stunden-Takt bevorzugt.

2. Schallemissionsmessung: Diese Messung erfolgt kontinuierlich. So wird mit den als Schallemissionssensoren betriebenen Aufnehmern 400 eine weitestgehend ständige Überwachung der Probenkörper gewährleistet. Sobald ein Signal (durch Rissbildung in der Probe) am Sensor registriert wird, wird dieses aufgezeichnet und einige Signalmerkmale werden automatisch analysiert. Die Signalabtastrate beträgt dazu beispielsweise mindestens 5 MHz.

3. Ultraschallmessung: Die Ultraschallmessung kann beispielsweise im Takt von 4 h bis beispielsweise einmal aller 12 h erfolgen. Die verwendeten SE-Wandler erlauben einen automatischen, zeitgetakteten Selbsttest. So kann die Erfassung der Ultraschallgeschwindigkeit einer Probe über lange Zeiträume hinweg zuverlässig automatisch erfolgen.

Nach einer Ausführungsform wird in einem geeigneten Mess-Raum, etwa einer geschlossenen doppelwandigen thermoisolierten Messkammer 100, das für die künstliche Bewitterung der Probekörper jeweils gewünschte Klima eingestellt. Wie vorstehend erläutert, kann für die Prüfung der Alkali-Kieselsäure-Reaktion an Betonen das Klima im Proberaum, bzw. der Probekammer 100 auf 40 °C und ≥ 99% Luftfeuchte, oder auf 60 °C und ≥ 98 % Luftfeuchte eingestellt sein. In Abhängigkeit von der jeweiligen Prüfaufgabe können weitere Klima-Faktoren in die Bewitterung einbezogen werden.

Beispielsweise sind aus anderen Bereichen der Materialprüfung sogenannte Salzkammersprühtests bekannt. Nach einer Ausführungsform der Erfindung kann aber auch die Atmosphäre oder die Temperatur nach Wunsch, beispielsweise ein zeitliches Klimaprofil, vorgegeben werden. Die Regulation des Klimas wird über eine Mess- und Regel-Einheit (110) geführt. Dazu weisen spezielle, auf die jeweiligen klimatischen Faktoren abgestimmte Sensoren Ist-Werte des Kammer-Innenraums aus. Ein Beispiel für einen derartigen Sensor ist ein Thermopaar. Ein weiteres Beispiel für einen solchen Sensor ist ein Luftfeuchte-Messer oder ein spezieller Gassensor. In Figur 1 sind diese Mess-Punkte schematisch durch schwarze Punkte im Inneren der Kammer dargestellt. Die Sensoren übermitteln nach einem wählbaren Takt Messwerte an die Steuer- und Regel-Einheit (110). Die jeweiligen Ist-Werte können in der Steuer- und Regel-Einheit 110 mit voreingestellten Soll-Werten abgeglichen werden und beispielsweise Magnetventile oder Schalter ansteuern. Im Schema der Figur 1 sind diese Medienzuführungen sowie Heiz- und Kühlelemente zur Vereinfachung nicht dargestellt.

In der klimatisierten Kammer wird der jeweilige Probekörper über die jeweils gewünschten Zeitspannen bewittert. Eine derartige Bewitterung kann sowohl kontinuierlich als auch diskontinuierlich, etwa in thermischen Zyklen geführt werden. Die während der Bewitterung vom Wegaufnehmer 300, bzw. den Schallemissionssensoren bzw. Aufnehmern 400, gelieferten oder durch die Einheit zur Messmoden-Steuerung und Messwert-Aufnahme (500) abgerufene Daten werden gespeichert, gegebenenfalls miteinander verrechnet und nach geeigneter Aufbereitung mit Hilfe einer Auswerte- und Ausgabe-Einheit zur Beurteilung des Probekörpers herangezogen.

Zur automatischen Messung der Längenänderung der Probenkörper mit einem geeigneten Wegaufnehmer 300 wurden die Beton-Probekörper vertikal im beschriebenen Rahmen 700 gehalten (Vgl. Fig. 2). Parallel dazu wird die Länge gesonderter, sonst aber identischer Probekörper einmal monatlich manuell gemäß den Vorgaben der Deutschen Alkali-Richtlinie gemessen.

Nach einer oder mehreren bevorzugten Ausführungsformen erfolgt die Messung der Schallgeschwindigkeit im Prüfkörper, bzw. die Messung der Ultraschallgeschwindigkeit. Die Erfassung akustischer Emissionen der Probe mit ein und denselben SE-Wandlern, beispielsweise piezoelektrischen Wandlern, wird während der Messung der Ultraschallgeschwindigkeit unterbrochen.

### Beispiel 1

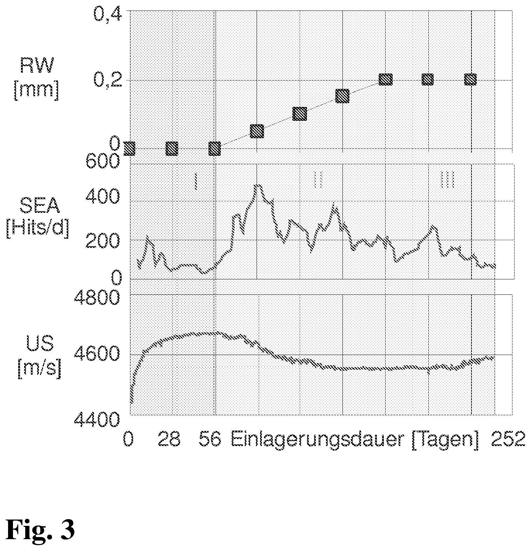

Aus einem Grauwackesplitt - Beton wurden zylindrische Probekörper einer Länge von 280 mm mit einem Durchmesser von 70 mm sowie würfelförmige Probekörper der Kantenlänge 300 mm hergestellt. Direkt nach dem Ausschalen der einen Tag zuvor hergestellten Probekörper wurden diese jeweils bei 40 °C und 100 % relativer Luftfeuchte in einer Klimakammer eingelagert. Ein Probekörper wurde im beschriebenen Messrahmen 700 (Vgl. Figur 2) gehalten und ist wie beschrieben mit je einem Wegaufnehmer 300 und zwei SE-Wandlern 400 bestückt. Der Würfel diente zur manuell vorgenommenen Messung der Rissweite gemäß den Vorschriften der "Alkali-Richtlinie". Die zusätzlich gewonnenen Ergebnisse der kontinuierlichen Messung von Ultraschallgeschwindigkeit (US) und Schallemissionsanalyse (SEA) an einem Grauwackesplitt-Beton mit einem Wasser/Zement-Wert (w/z) von 0,35 sind in Figur 3 der Rissweite (RW) gegenübergestellt.

Die gute Korrelation der Kurvenverläufe ist augenfällig. Es ist erstmals möglich, die Hydratations- und Schädigungsmechanismen während der ersten Tage einer 40 °C Lagerung drei gesonderten Phasen zuzuordnen. Zu Beginn (Phase I) ist eine Steigerung der Ultraschallgeschwindigkeit durch die zunehmende Hydratation des Beton nachweisbar. In Phase II weist eine starke Zunahme der Längenänderung mit zahlreichen akustischen Emissionen und einer Reduktion oder Stagnation der Ultraschalllaufzeit auf eine Rissbildung hin. Wenn die Dehnung des Betons durch die AKR fast abgeschlossen ist (Phase III), sind nur noch wenige akustische Ereignisse nachweisbar. Vermutlich durch die Füllung entstandener Risse mit Gel erfolgt ein erneuter Anstieg der integralen Ultraschallgesch windigkeit.

### Beispiel 2

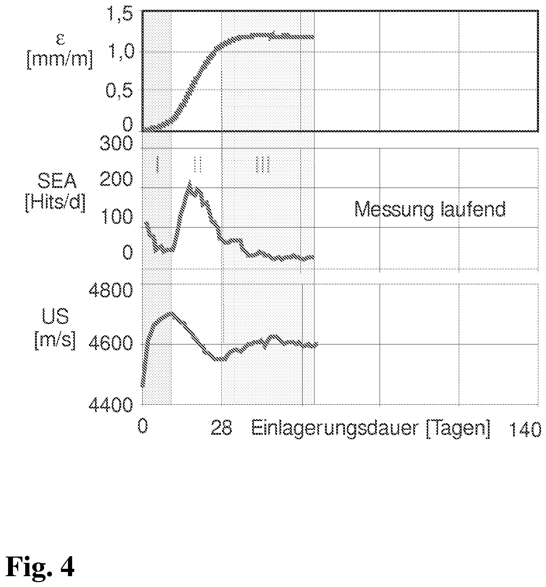

In Analogie zu Beispiel 1 zeigt Figur 4 erste Ergebnisse der Lagerung des mit Grauwackesplitt bei w/z = 0,35 hergestellten Betons bei 60 °C und ≥ 98 % relativer Luftfeuchte, d.h. im 60 °C Betonversuch. Auch hier sind drei Phasen erkennbar: I - Hydratation, II - Rissbildung, III - vermutlich das Verfüllen der Risse mit AKR-Gel.

### Beispiel 3

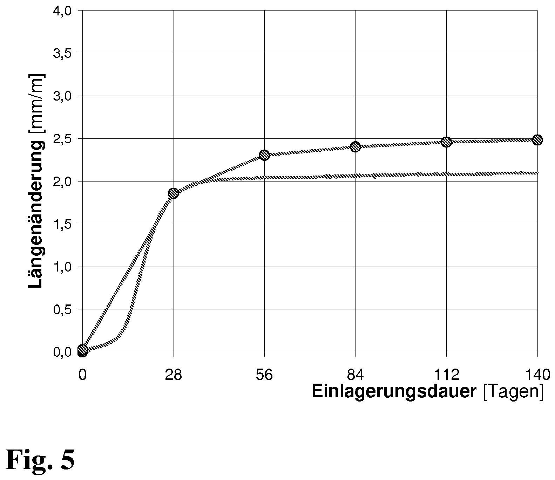

Die Vorteile des hier vorgestellten Messverfahrens unter Verwendung der beschriebenen Messanordnung im Vergleich zu einer manuell durchgeführten diskontinuierlichen Messung und zwangsläufig unterbrochener Bewitterung bzw. Klimatisierung des Prüfkörpers sind in Figur 5 dargestellt. Die Bewitterung erfolgte hier bei 60 °C und ≥ 98 % realtiver Luftfeuchte. Der mit den schwarz markierten Messpunkten (●) versehene Verlauf entspricht der manuellen Dehnungsmessung. Die als durchgehende Linie ohne markierte Messpunkte dargestellte Mess-Kurve entspricht der automatisch und bei kontinuierlicher Bewitterung bzw. Klimatisierung des Probekörpers ermittelten Längenänderung, beide dargestellt in mm/m. Der zylindrische Probenkörper hatte auch hier einen Durchmesser Ø von 70 mm und eine Länge L von 280 mm.

### Beispiel 4

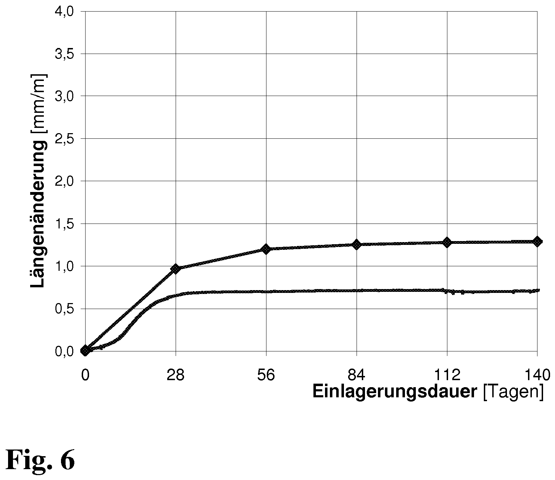

In Figur 6 sind zum Vergleich die entsprechenden Kurvenverläufe für eine weitere Gesteinskörnung, für oberrheinischen Kiesedelsplitt, dargestellt. Wie für Beispiel 3 beschrieben, erfolgte die Messung der Längenänderung kontinuierlich und automatisch. Es wurden Probekörper (Ø 70 mm, L 280 mm) mit Kiesedelsplitt vom Oberrhein bei w/z = 0,35 hergestellt und im 60 °C Betonversuch (60 °C, ≥ 98 % Luftfeuchte) AKRprovozierend gelagert. Dargestellt ist die Längenänderung in mm/m als Vergleich von diskontinuierlicher, manueller Messung unter Entnahme des Probekörpers aus der Klimakammer (Kurvenverlauf mit schwarzen markierten Messpunkten (●)) mit der automatischen, kontinuierlichen Messung (durchgezogene flachere Kurve).

### Beispiel 5

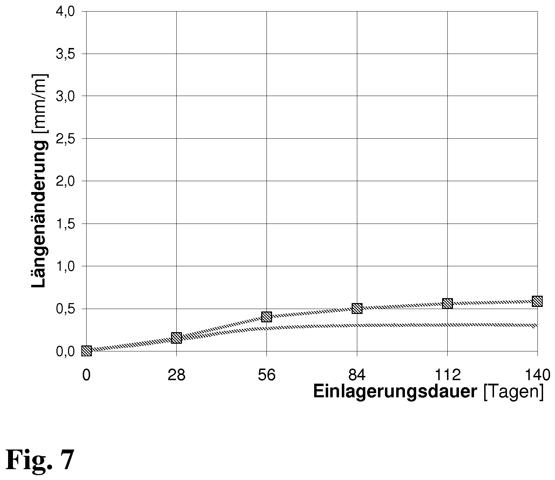

Figur 7 zeigt die mit den Beispielen 3 und 4 vergleichbaren Messungen an Beton-Probekörpern, die mit gebrochenem Rhyolith bei w/z = 0,35 hergestellt wurden. Auch hier stellt der mit den schwarz markierten Messpunkten (●) versehene Verlauf die manuell und diskontinuierlich ermittelten Messwerte, die als durchgehende Linie ohne markierte Messpunkte dargestellte Mess-Kurve den automatisch und kontinuierlich gemessenen Verlauf dar. Die Größe der Probenkörper war zu denen in den Beispielen 3 und 4 identisch.

Die Grauwacke-Proben zeigten erwartungsgemäß die höchste Dehnung, während die Dehnung der Rhyolith-Proben sehr gering ist. Wie im Vergleich zum Nebelkammer-Test ersichtlich, sind die zu beobachtenden Tendenzen einander ähnlich, jedoch ist die Dehnung im 60 °C-Betonversuch deutlich größer als in der 40 °C Nebelkammer.

Die kontinuierlich gemessenen Dehnungswerte sind bei allen Proben (Beispiele 3, 4 und 5) geringer, als die manuell gemäß Alkali-Richtlinie ermittelten. Die genauen Ursachen für diese Diskrepanz bleiben zunächst unklar, sind aber offenbar in der zwischenzeitlichen Abkühlung der Proben von 60 °C auf die Messtemperatur von 20 °C zu suchen. Temperaturbedingte Unterschiede der Viskosität des gebildeten Kieselgels lassen ein unterschiedlich rasches Eindringen der Gele in Kapillarräume vermuten. Daraus ergeben sich unterschiedlich starke Konzentrationsgradienten und durch den Temperaturwechsel ausgelöste innere Spannungen. Ein weiterer Grund mag im verstärkten Auslaugen der Proben während der kontinuierlichen Lagerung liegen.

Aus der Korrelation von Messdaten der mechanischen Dehnungsmessung an einem Probekörper, beispielsweise mit Hilfe eines induktiven Wegaufnehmers, mit Daten einer zeitgleich vorgenommenen akustischen Emissions-Messung am gleichen Probekörper ergeben sich Vorteile hinsichtlich der Präzision und Zuverlässigkeit der erhaltenen Messwerte. Für eine kombinierte Messung werden zwei SE-Sensoren, beispielsweise piezoelektrische Wandler, wie beschrieben an gegenüberliegenden Stirnflächen des Probekörpers, platziert. Auf einer der beiden Stirnflächen des Probekörpers sitzt neben einem der SE-Sensoren 400 der Messfühler des Wegaufnehmers 300 auf.

Durch die präzise Bestimmung der Ankunftszeit von Schallemissionssignalen an den beiden SE-Sensoren kann der Ursprung des Signals lokalisiert werden. Somit ist eine Lokalisation der Rissbildung im Probekörper möglich.

Da sich naturgemäß die Schallgeschwindigkeit im Feststoff (Zement bzw. Gesteinskörnung) und im gebildeten Kieselgel unterscheidet, kann der Verlauf der mit der AKR ausgelösten chemischen und (mikro-)strukturellen Veränderungen im Probekörper während der klimatisierten Lagerung verfolgt werden.

Die hier beschriebene bisher einzigartige Kombination zerstörungsfreier Prüfverfahren am Beispiel einer kontinuierlichen Längenmessung, der Registrierung akustischer Emissionen und der Messung der Ultraschallgeschwindigkeit (Schallgeschwindigkeit) gestattet es, die Phasen der Hydratation der Betonproben von denen der eigentlichen Schadensreaktion zeitlich zu trennen. Die beschriebenen Messprinzipien lassen sich mit weiteren diskontinuierlichen Messungen kombinieren. So kann beispielsweise die Mikro-3D-Computertomographie zur räumlichen Visualisierung der Risse herangezogen werden.

Die vorliegende Erfindung wurde anhand von Ausführungsbeispielen erläutert. Diese Ausführungsbeispiele sollten keinesfalls als einschränkend für die vorliegende Erfindung verstanden werden. Die Erfindung ist in den folgenden Ansprüchen definiert.

### BEZUGSZEICHENLISTE

- 100: Klimatisierter Messraum / Messkammer
- 110: Einheit zur Messung und Regelung von Klimadaten
- 200: Probekörper
- 300: Wegaufnehmer
- 400: SE-Sensor
- 500: Einheit zur Messmoden-Steuerung und Messwert-Aufnahme
- 600: Bügel
- 650: Gummipuffer
- 700: Rahmen
- 710: Säule
- 720: oberes Querhaupt
- 730: Basis
- 740: Befestigungsmittel
- I: Phase der Hydratation
- II: Phase der Rissbildung
- III: Phase der Gelbildung in den Rissen

## Patentansprüche

1. Messeinrichtung, umfassend
- eine Klimakammer (100) mit einem Rahmen (700) zur Aufnahme eines Probekörpers (200),
- einen Wegaufnehmer (300), der in der Klimakammer (100) angeordnet ist, sowie
- zwei Schallemissionssensoren (400), die an gegenüberliegenden Stirnflächen des Probekörpers (200) ansetzbar sind, wobei der Rahmen so ausgeführt ist, dass der Wegaufnehmer (300) neben einem der Schallemissionssensoren (400) aufsetzbar ist.

2. Messeinrichtung nach Anspruch 1, wobei
der Wegaufnehmer (300) ausgewählt ist aus induktiven, kapazitiven, oder optischen Wegaufnehmern.

3. Messeinrichtung nach Anspruch 1 oder 2, weiterhin umfassend eine Steuer- und Auswerteeinheit (500), wobei die Steuer- und Auswerteeinheit (500) eingerichtet ist, die Signale des Wegaufnehmers (300) kontinuierlich oder in vorgegebenen Zeitabständen aufzunehmen.

4. Messverfahren für einen Probekörper (200), umfassend,
- Klimatisieren des Probekörpers (200); und
- Messung einer Längeänderung des Probekörpers (200) unter Beibehaltung der Klimatisierung; und.
- zeitgleiche akustische Emissionsmessung am Probekörper (200).

5. Messverfahren nach Anspruch 4, wobei
die Längenmessung mit einem induktiven Wegaufnehmer (300) erfolgt.

6. Messverfahren nach Anspruch 3, wobei
der Probekörper (200) ein Betonkörper ist; und wobei das Klimatisieren geeignet ist, die Alkali-Kieselsäure-Reaktion des Probekörpers (200) zu provozieren.

7. Messverfahren nach Anspruch 3 oder 4, wobei
das Klimatisieren und/oder die Messung automatisch erfolgen.

## Claims

1. A measuring device comprising
- an air-conditioning chamber (100) with a frame (700) for accommodating a sample (200),
- a displacement gauge (300) which is arranged in the air-conditioning chamber (100) as well as
- two sound emission sensors (400) which can be placed on opposite faces of the sample (200), wherein the frame is designed so that the displacement gauge (300) can be placed next to one of the sound emission sensors (400).

2. The measuring device according to claim 1, wherein
the displacement gauge (300) is selected from inductive, capacitive or optical displacement gauges.

3. The measuring device according to claim 1 or 2, further comprising
a control and evaluation unit (500), wherein the control and evaluation unit (500) is adapted to record the signals of the displacement gauge (300) continuously or at predefined time intervals.

4. A measurement method for a sample (200), comprising
- air conditioning the sample (200); and
- measuring a length variation of the sample (200) whilst maintaining the air conditioning; and
- simultaneous acoustic emission measurement on the sample (200).

5. The measurement method according to claim 4, wherein
the length measurement is made using an inductive displacement gauge (300).

6. The measurement method according to claim 3, wherein
the sample (200) is a concrete body; and wherein the air conditioning is suitable for provoking an alkali-silicic acid region of the sample (200).

7. The measurement method according to claim 3 or 4, wherein
the air conditioning and/or the measurement take place automatically.

## Revendications

1. Dispositif de mesure comprenant
- une chambre climatisée (100) avec un cadre (700) pour recevoir un échantillon d'essai (200),
- un capteur de déplacement (300), qui est disposé dans la chambre climatisée (100), et
- deux détecteurs d'émission acoustique (400), qui peuvent être installés au niveau de surfaces frontales opposées de l'échantillon d'essai (200), où le cadre est réalisé de telle manière que le capteur de déplacement (300) peut être installé à côté de l'un des détecteurs d'émission acoustique (400).

2. Dispositif de mesure selon la revendication 1, où
le capteur de déplacement (300) est choisi parmi les capteurs de déplacement inductifs, capacitifs ou optiques.

3. Dispositif de mesure selon la revendication 1 ou 2, comprenant en outre
une unité de commande et d'exploitation (500), où l'unité de commande et d'exploitation (500) est agencée pour recevoir les signaux du capteur de déplacement (300) de manière continue ou à des intervalles de temps prédéterminés.

4. Procédé de mesure pour un échantillon d'essai (200), comprenant
- la climatisation de l'échantillon d'essai (200) ; et
- la mesure d'une variation de longueur de l'échantillon d'essai (200) avec maintien de la climatisation ; et
- la mesure d'émission acoustique simultanée au niveau de l'échantillon d'essai (200).

5. Procédé de mesure selon la revendication 4, où
la mesure de longueur a lieu avec un capteur de déplacement (300) inductif.

6. Procédé de mesure selon la revendication 3, où
l'échantillon d'essai (200) est un échantillon de béton ; et où la climatisation est appropriée pour provoquer la réaction alcalis-acide silicique de l'échantillon d'essai (200).

7. Procédé de mesure selon la revendication 3 ou 4, où la climatisation et/ou la mesure ont lieu automatiquement.
